**Europäisches Patentamt**

**European Patent Office**

**Office européen des brevets**

(19)

(11) Veröffentlichungsnummer : **0 491 147 B1**

(12)

# EUROPÄISCHE PATENTSCHRIFT

(45) Veröffentlichungstag der Patentschrift :
**12.05.93 Patentblatt 93/19**

(51) Int. Cl.$^5$ : **C07F 1/12**, C03C 17/44

(21) Anmeldenummer : **91118599.9**

(22) Anmeldetag : **31.10.91**

(54) **Verfahren zur Herstellung von Gold(I)mercaptiden.**

(30) Priorität : **18.12.90 DE 4040447**

(43) Veröffentlichungstag der Anmeldung :
**24.06.92 Patentblatt 92/26**

(45) Bekanntmachung des Hinweises auf die
Patenterteilung :
**12.05.93 Patentblatt 93/19**

(84) Benannte Vertragsstaaten :
**AT BE CH DE DK ES FR GB IT LI NL SE**

(56) Entgegenhaltungen :
**DE-B- 1 286 866**

(56) Entgegenhaltungen :
**CHEMICAL ABSTRACTS, vol. 84, no. 6, 9. Februar 1976, Columbus, Ohio, US; abstract no. 35807E, G. NATILE ET AL: 'Chloroauric acid as oxidant. Stereospecific oxidation of methionine to methionine sulfoxide.' Seite 337 ;Spalte 2 ;**
**CHEMICAL ABSTRACTS, vol. 111, no. 4, 24. Juli 1989, Columbus, Ohio, US; abstract no. 32688G, A. A. DANOPOULOS ET AL: 'Complexes of amino acids with gold.' Seite 633 ;Spalte 1 ;**

(73) Patentinhaber : **Degussa Aktiengesellschaft**
**Weissfrauenstrasse 9**
**W-6000 Frankfurt am Main 1 (DE)**

(72) Erfinder : **Lotze, Marion, Dr.**
**Am Galgenberg 18**
**W-6451 Hammersbach (DE)**
Erfinder : **Bauer, Margit**
**Limesstrasse 11**
**W-6365 Nidderau 5 (DE)**

## Beschreibung

Die Erfindung richtet sich auf ein Verfahren zur Herstellung von Gold(I)mercaptiden der allgemeinen Formel Au-S-R durch Umsetzung eines aus einem Gold(III)halogenid oder Gold(III)halogenkomplex und einem Dialkylsulfid erhältlichen Gold(I)halogenid-dialkylsulfidkomplexes mit einer Mercaptoverbindung der Formel R-S-H.

Aliphatische, cycloaliphatische und aromatische Gold(I)mercaptide der allgemeinen Formel Au-S-R, worin R für einen organischen Rest steht, wobei der Schwefel an ein aliphatisches, cycloaliphatisches oder aromatisches C-Atom des Rests R gebunden ist, sind lange bekannt. Sie finden Verwendung in sogenannten Goldpräparaten zum Vergolden fester, insbesondere keramischer, Materialien, als Bestandteil in Lüsterpräparaten und zur Herstellung von Leiterbahnen in integrierten Schaltkreisen. Eine Übersicht über die Herstellungsverfahren der Gold(I)mercaptide sowie ihre Verwendung vermitteln die DE-PS 12 84 808, DE-AS 12 86 866, DE-AS 12 98 828 und US-PS 4,221,826.

Zur Herstellung der Gold(I)mercaptide der Formel R-S-Au sind hiernach zwei Verfahren bekannt:

$$(A) \qquad 3\ RSH + AuX_3 \rightarrow RSAu + R\text{-}S\text{-}S\text{-}R + 3\ HX$$

$$(B) \qquad 2\ R'_2S + AuX_3 + H_2O \rightarrow AuX \cdot R'_2S + R'_2SO + 2\ HX \quad AuX \cdot R'_2S + RSH \rightarrow RSAu + R'_2S + HX$$

Verfahren (A) beinhaltet die Reaktion eines Gold(III)halogenids mit drei Äquivalenten Mercaptan in Gegenwart eines organischen Lösungsmittels. Das Gold(III)halogenid wird durch zwei Äquivalente Mercaptan unter Disulfidbildung zum Gold(I)halogenid reduziert, das sich in Gegenwart des dritten Äquivalents Mercaptan sofort zum Gold(I)mercaptid umsetzt.

Das Verfahren (A) disqualifiziert sich durch den Verlust von zwei Äquivalenten Mercaptan. Die Mercaptane sind in der Regel auf ihren Anwendungszweck ausgerichtete chemisch maßgeschneiderte Verbindungen und somit als Reduktionsmittel zu kostspielig. Hinzu kommt, daß die Trennung des Gold(I)mercaptids vom Disulfid sehr aufwendig ist, große Mengen organische Lösungsmittel erfordert und häufig zu Ausbeuteverlusten führt.

Beim Verfahren (B) wird die Reduktion des Gold(III)halogenids und seine Komplexbildung in Gegenwart von mindestens einem Äquivalent Wasser und zwei Äquivalenten eines Dialkylsulfids durchgeführt. Die gebildete Gold(I)halogenid-dialkylsulfid-Komplexverbindung reagiert mit dem Mercaptan zum Gold(I)mercaptid unter Freisetzung des Dialkylsulfids. Als Nebenprodukt der Reduktionsstufe fällt ein Äquivalent Dialkylsulfoxid an.

Im Verfahren (B) kann ein gegenüber dem maßgeschneiderten Mercaptan des Verfahrens (A) preisgünstigeres Dialkylsulfid, wie Dimethyl-, Diethyl-, Dibutyl- oder Dioctylsulfid als Reduktionsmittel verwendet werden. Bei diesem Verfahren (B) enthält das Reaktionsgemisch nach der Umsetzung pro Äquivalent Gold(I)mercaptid üblicherweise ein bis zwei Äquivalente Dialkylsulfid und ein Äquivalent Dialkylsulfoxid an, die vom Gold(I)mercaptid abgetrennt werden müssen.

Nachteilig an dem Verfahren (B) ist, daß die bisher verwendeten Dialkylsulfide wegen ihres penetranten Geruchs aus arbeitshygienischer Sicht sehr kritisch beurteilt werden. Auch in den erzeugten Gold(I)mercaptiden führen Spuren noch verbliebenen Dialkylsulfids zu erheblichen Geruchsproblemen.

Während die Sulfoxide der vorgenannten Dialkylsulfide ausreichend wasserlöslich sind und sich bei der Aufarbeitung der Reaktionsgemische von den Gold(I)mercaptiden im allgemeinen befriedigend abtrennen lassen, sind die Dialkylsulfide selbst wasserunlöslich. Die Dialkylsulfide müssen daher durch organische Lösungsmittel vom Gold(I)mercaptid getrennt werden. Diese Lösungsmittel müssen sorgfältig gewählt werden, da sie nur das Dialkylsulfid, nicht aber das Gold(I)mercaptid lösen dürfen. Die Dialkylsulfide sind zumeist, wie die Beispiele in oben genannten Patentschriften ausweisen, nicht vollständig durch Auswaschen der zumeist festen Gold(I)mercaptide abzutrennen. Es bedarf in den meisten Fällen einer Umfällung. Eine solche Umfällung aber beschränkt sich auf in organischen Lösungsmitteln lösliche Gold(I)mercaptide.

Im Bestreben, das vorbekannte Verfahren zu verbessern, wurden von der Anmelderin der vorliegenden Erfindung auch wasserlösliche Dialkylsulfide, wie Bis-hydroxyethylsulfid und 2,2'-Thiodiessigsäure, im infragestehenden Verfahren eingesetzt. Es zeigte sich hierbei, daß die gewünschten Umsetzungen zwar stattfinden, die Löslichkeit der genannten Sulfide in organischen Lösungsmitteln, wie Dichlormethan, Toluol, Aceton und Ethanol, aber so groß ist, daß im Falle der Herstellung, Isolierung und Reinigung von gut löslichen Gold(I)mercaptiden in Gegenwart organischer Lösungsmittel die vorerwähnten Probleme auftreten.

Aufgabe der Erfindung ist, das Verfahren gemäß dem Reaktionsprinzip (B) so zu verbessern, daß das gebildete Gold(I)mercaptid unabhängig von seiner Struktur und Löslichkeit in einfacher Weise und vollständig von dem eingesetzten Dialkylsulfid und entsprechenden Dialkylsulfoxid abgetrennt werden kann. Eine weitere Aufgabe besteht darin, ein möglichst geruchsarmes Dialkylsulfid aufzufinden, das zur erforderlichen Komplexbildung und Gold(I)mercaptidbildung in möglichst quantitativer Ausbeute befähigt ist. Schließlich sollten der technische Aufwand und die Menge an organischen Lösungsmitteln für die Aufarbeitung des Reaktionsgemischs und Reinigung des Gold(I)mercaptids möglichst gering sein.

Die Aufgabe wird gelöst durch ein Verfahren zur Herstellung von Gold(I)mercaptiden der allgemeinen Formel Au-S-R, wobei R ein organischer Rest ist, durch Umsetzung eines Gold(III)halogenids oder Gold(III)halogenkomplexes mit einem Dialkylsulfid im Molverhältnis von 1 zu gleich/größer 2 in Gegenwart einer zur Au(III)verbindung mindestens äquivalenten Menge Wasser bei -20 bis 40 °C unter Bildung des Gold(I)halogenid-dialkylsulfidkomplexes und Dialkylsulfoxids, Umsetzung des Gold(I)halogenid-dialkylsulfidkomplexes bei -20 bis 40 °C und bei Bedarf in Anwesenheit eines organischen Lösungsmittels oder Lösungsmittelgemisches mit einem Mercaptan der Formel RSH, wobei R die gleiche Bedeutung hat wie in den Gold(I)mercaptiden, und Isolierung des Gold(I)mercaptids aus dem Reaktionsgemisch in an sich bekannter Weise, das dadurch gekennzeichnet ist, daß man als Dialkylsulfid eine ($C_1$- bis $C_8$-)Alkylmercapto-($C_3$- bis $C_6$-)aminosäure, wobei die Alkylmercapto- und die Aminogruppe an verschiedenen C-Atomen gebunden sind, oder ein wasserlösliches Salz hiervon verwendet.

Die erfindungsgemäß zu verwendenden Dialkylsulfide tragen die Alkylmercaptogruppe und Aminogruppe an unterschiedlichen C-Atomen der Carbonsäure, welche 3 bis 6 C-Atome aufweist und linear oder verzweigt sein kann. Die Aminogruppe kann zur Carboxylgruppe nachbarständig oder durch mehrere C-Atome getrennt sein; gleiches gilt für die Alkylmercaptogruppe hinsichtlich der Carboxyl- und Aminogruppe. Die Alkylgruppe der Alkylmercaptogruppe kann linear oder verzweigt sein und 1 bis 8 C-Atome aufweisen; vorzugsweise handelt es sich um $C_1$- bis $C_4$-Alkyl, insbesondere Methyl. Die Aminosäuregruppe der bevorzugten Dialkylsulfide enthält 3 oder 4 C-Atome. Aufgrund ihrer leichten Verfügbarkeit sind S-alkylisiertes Cystein und insbesondere Methionin bevorzugte Dialkylsulfide; vorzugsweise werden diese Alkylmercaptoaminosäuren in der D,L-Form eingesetzt.

Unter den Salzen der erfindungsgemäß zu verwendenden Dialkylsulfide werden insbesondere Salze der Alkylmercaptoaminosäuren mit starken Säuren, wie z. B. Halogenwasserstoffsäure, Schwefelsäure, Phosphorsäure, Perchlorsäure, verstanden; solche Salze, die Hydrochloride werden bevorzugt, führen zu stabilen Gold(I)halogenid-dialkylsulfid-komplexen. Im Prinzip können auch Alkali- bzw. Ammoniumsalze der Alkylmercaptoaminosäure Einsatz finden.

Unter den Gold(I)mercaptiden der allgemeinen Formel R-S-Au werden monomere und aggregierte Formen der Gold(I)mercaptide verstanden. Das Schwefelatom ist an ein aliphatisches, cycloaliphatisches oder aromatisches C-Atom des organischen Rests R, der ein oder mehrere funktionelle Gruppen, wie beispielsweise Ester-, Hydroxyl-, Ether- oder olefinische Gruppen, aufweisen kann, gebunden. Beispielhafte Gold(I)mercaptide sind in den eingangs zitierten Dokumenten genannt.

Als Gold(III)halogenid kommen $AuCl_3$, $AuBr_3$ und $AuJ_3$ zum Einsatz; unter den Gold(III)halogenkomplexen werden $HAuCl_4$ (Tetrachlorogoldsäure), $HAuBr_4$ bzw. $HAuJ_4$ bevorzugt, wobei die Tetrachlorogoldsäure besonders geeignet ist. Verwendung finden können auch Komplexe der genannten Au(III)halogenide mit einem neutralen Liganden, wie beispielsweise einem tertiären Amin oder tertiären Phosphin.

Im allgemeinen werden die Stufe der Bildung des Gold(I)halogenid-dialkylsulfidkomplexes und die Umsetzung mit dem Mercaptan der Formel RSH hintereinander ausgeführt. Vorzugsweise verwendet man in der ersten Stufe Wasser als Lösungsmittel und setzt dem erhaltenen Reaktionsgemisch, das den Gold(I)halogenid-dialkylsulfid-komplex in gelöster und ggf. auch suspendierter Form enthält, das Mercaptan, pur oder in einem organischen Lösungsmittel oder Lösungsmittelgemisch gelöst, zu. In ausgewählten Fällen können die beiden Verfahrensstufen auch parallel nebeneinander durchgeführt werden, wie dies in der DE-AS 12 98 828 vorgeschlagen wurde; diese Ausführungsform setzt voraus, daß das eingesetzte Mercaptan praktisch nicht in der Weise des Verfahrensprinzips des Formelchemas (A) reagiert.

Obgleich das Dialkylsulfid im Überschuß eingesetzt werden kann, werden aus wirtschaftlichen Erwägungen pro Mol Au(III)halogenid bzw. Au(III)halogenkomplex meist nur zwei Mol Dialkylsulfid verwendet. Das Mercaptan wird in der Regel in äquimolarer Menge zum Gold eingesetzt, obgleich ein Überschuß an Mercaptan nicht ausgeschlossen wird.

Beide Reaktionsstufen werden im Temperaturbereich zwischen -20 und 40 °C, vorzugsweise zwiswchen 0 und 20 °C, ausgeführt, wobei die Temperatur in den beiden Stufen nicht identisch zu sein braucht.

Zur Herstellung von in organischen Lösungsmitteln schwerlöslichen Gold(I)mercaptiden, wie beispielsweise Gold(I)isopropylmercaptid, wird die zweite Reaktionsstufe vorzugsweise entweder in Abwesenheit eines organischen Lösungsmittels oder in Gegenwart eines mit dem Wasser aus der ersten Reaktionsstufe eine einheitliche flüssige Phase bildenden organischen Lösungsmittelgemischs aus einem mit Wasser nicht mischbaren und einem mit Wasser mischbaren Lösungsmittel. Unter den mit Wasser nicht mischbaren Lösungsmitteln sind aromatische und aliphatische Kohlenwasserstoffe, chlorierte Kohlenwasserstoffe und Ether hervorzuheben, unter den mit Wasser mischbaren Lösungsmitteln $C_1$- bis $C_4$-Alkohole, $C_3$- bis $C_5$-Ketone, N-Alkylpyrrolidone und Dimethylsulfoxid. Das aus der klaren Reaktionslösung ausgefallene Gold(I)mercaptid wird durch Filtrieren oder Zentrifugieren vom flüssigen Reaktionsmedium abgetrennt, gewaschen und im allgemeinen unter 50 °C getrocknet.

In organischen Lösungsmitteln gut lösliche Gold(I)mercaptide wie beispielsweise Gold(I)tert.-dodecylmercapid werden vorzugsweise in Gegenwart von wasserunlöslichen organischen Lösungsmitteln aus der vorstehend genannten Reihe hergestellt. Die Umsetzung erfolgt damit im Zweiphasensystem, und das in der organischen Phase gelöste Gold(I)mercaptid läßt sich durch Entfernen des Lösungsmittels gewinnen.

Aus J. Chem. Soc. Chem. Comm. 1973, 878 ist die stereospezifische Oxidation von D- bzw. L-Methionin zum entsprechenden Sulfoxid mittels Tetrachlorogoldsäure bekannt. Hinweise, Methionin als Dialkylsulfid in dem erfindungsgemäßen Verfahren zur Herstellung von Gold(I)mercaptiden einzusetzen, können diesem Dokument nicht entnommen werden.

Es war nicht vorhersehbar, daß die anspruchsgemäßen Alkylmercaptoaminosäuren stabile Gold(I)halogenid-Komplexe bilden, die mit Mercaptanen unterschiedlichster Struktur in hoher Ausbeute und hoher Raum-Zeit-Ausbeute zu Gold(I)mercaptiden umgesetzt werden können. Ein herausragender Vorteil der Alkylmercaptoaminosäuren besteht in ihrer Wasserlöslichkeit einerseits und Unlöslichkeit in organischen Lösungsmitteln andererseits. Hierdurch lassen sich die Gold(I)mercaptide in einfacher Weise von der freigesetzten Alklymercaptoaminosäure sowie dessen Sulfoxid abtrennen und reinigen. Der bisher erforderliche Aufwand zur Reinigung sowie die erforderliche Lösungsmittelmenge konnten damit vermindert und die Reinheit des Gold(I)mercaptids sowie die Ausbeute und Raum-Zeit-Ausbeute erhöht werden. Ein weiterer Vorteil besteht in dem mäßigen Geruch der Alkylmercaptoaminosäuren, der ein Arbeiten ohne Geruchsbelästung erlaubt. Das erfindungsgemäße Verfahren läßt sich unabhängig von der Struktur und Löslichkeit der Gold(I)mercaptide mit gutem Erfolg zur Herstellung derselben anwenden.

**Beispiel 1 und Vergleichsbeispiel 1**

Herstellung von Gold(I)isopropylmercaptid

Die Reduktion und Komplexbildung wurde in Wasser durchgeführt, indem eine wäßrige Lösung Tetrachlorogoldsäure bei 5 °C zu einer Lösung bzw. Emulsion des Dialkylsulfids zugetropft wurde. Anschließend wurde das Mercaptan zugetropft. Die Ansatzmengen und Ergebnisse sowie Maßnahmen zur Reinigung folgen aus der Tabelle:

| Beispiel 1 | Vergleichsbeispiel 1 gemäß DE-AS 12 86 866 |
|---|---|

1. Stufe:

| 59,7 g (0,4 Mol) D,L-Methionin | 54,0 g (0,6 Mol) Diäthylsulfid |
|---|---|
| 68,0 g (0,2 Mol) HAuCl$_4$ | 68,0 g (0,2 Mol) HAuCl$_4$ |
| 1000 g Wasser | 1000 g Wasser |

2. Stufe:

| 15,2 g (0,2 Mol) 2-Propanthiol | 16,8 g (0,22 Mol) 2-Propanthiol |
|---|---|
| 20,0 g Dichlormethan | |
| 20,0 g Ethanol | |

Reinigung:

| 1 x Waschen mit 100 ml Ethanol | 3 x Waschen mit je 300 ml Wasser 2 x Waschen mit je 500 ml Methanol 1 x Waschen mit je 300 ml Methanol |
|---|---|
| Au-Gehalt: 72,4 % | 72,4 % |
| Ausbeute: 89,0 % (bezogen auf umgesetztes Gold) | 74,0 % |

**Beispiel 2 und Vergleichsbeispiel 2**

Herstellung von Gold(I)tert.-dodecylmercaptid

Die Verfahrensführung entspricht derjenigen von Beispiel 1 und Vergleichsbeispiel 1. Die Ansetzungen, Maßnahmen zur Reinigung und Ergebnisse folgen aus der Tabelle:

5

| Beispiel 2 | Vergleichsbeispiel 2 gemäß DE-AS 12 98 828 |
|---|---|

1.Stufe:

| 59,7 g (0,4 Mol) D,L-Methionin | 37,3 g (0,6 Mol) Dimethylsulfid |
|---|---|
| 68,0 g (0,2 Mol) HAuCl$_4$ | 60,7 g (0,2 Mol) AuCl$_3$ |
| 1000 g Wasser | 1000 g Wasser |

2. Stufe:

| 43,5 g (0,2 Mol) tert.-Dodecylmercaptan | 43,5 g (0,2 Mol) tert.-Dodecylmercaptan |
|---|---|
| 100 g Dichlormethan | 30 g Chloroform |

Reinigung:

| keine (nur Abdestillieren des CH$_2$Cl$_2$) | 3 x Waschen mit je 300 ml Wasser bei 50 $^\circ$C 1 x Waschen mit 150 ml Methanol 1 x Umfällen durch Lösen in 50 ml Chloroform und Ausfällen mit 1200 ml Methanol |
|---|---|
| Au-Gehalt: 48,6 % | 48,2 % |
| Ausbeute: 98,0 % | 97,0 % |

**Patentansprüche**

1. Verfahren zur Herstellung von Gold(I)mercaptiden der allgemeinen Formel Au-S-R, wobei R ein organischer Rest ist, durch Umsetzung eines Gold(III)halogenids oder Gold(III)halogenkomplexes mit einem Dialkylsulfid im Molverhältnis von 1 zu gleich/größer 2 in Gegenwart einer zur Au(III)verbindung mindestens äquivalenten Menge Wasser bei -20 bis 40 °C unter Bildung des Gold(I)halogenid-dialkylsulfidkomplexes und Dialkylsulfoxids, Umsetzung des Gold(I)halogenid-dialkylsulfidkomplexes bei -20 bis 40 °C und bei Bedarf in Anwesenheit eines organischen Lösungsmittels oder Lösungsmittelgemisches mit einem Mercaptan der Formel RSH, wobei R die gleiche Bedeutung hat wie in den Gold(I)mercaptiden, und Isolierung des Gold(I)mercaptids aus dem Reaktionsgemisch in an sich bekannter Weise,
dadurch gekennzeichnet,
daß man als Dialkylsulfid eine (C$_1$- bis C$_8$-)Alkylmercapto-(C$_3$- bis C$_6$-)aminosäure, wobei die Alkylmercapto- und die Aminogruppe an verschiedenen C-Atomen gebunden sind, oder ein wasserlösliches Salz hiervon verwendet.

2. Verfahren nach Anspruch 1,
dadurch gekennzeichnet,
daß man als Dialkylsulfid eine Methylmercapto-(C$_3$- oder C$_4$-)aminosäure verwendet.

**3.** Verfahren nach Anspruch 1 oder 2,
dadurch gekennzeichnet,
daß man DL-Methionin verwendet.

**4.** Verfahren nach einem der Ansprüche 1 bis 4,
dadurch gekennzeichnet,
daß man zunächst die Reduktionsstufe in Wasser durchführt und das dabei erhaltene Reaktionsgemisch anschließend in Gegenwart eines organischen Lösungsmittels oder Lösungsmittelgemischs mit dem Mercaptan, vorzugsweise bei 0 bis 20 °C, umsetzt.

## Claims

**1.** A process for the production of gold(I) mercaptides corresponding to the general formula Au-S-R, in which R is an organic radical, by reaction of a gold(I) halide or gold(III)/halogen complex with a dialkyl sulfide in a molar ratio of 1:2 or 1:>2 in the presence of a quantity of water at least equivalent to the Au(III) compound at -20 to 40°C to form the gold(I) halide/dialkyl sulfide complex and dialkyl sulfoxide, reaction of the gold(I) halide/dialkyl sulfide complex at -20 to 40°C and, if necessary, in the presence of an organic solvent or solvent mixture with a mercaptan corresponding to the formula RSH, in which R has the same meaning as in the gold(I) mercaptides, and isolation of the gold(I) mercaptide from the reaction mixture by methods known <u>per se</u>, characterized in that a $(C_{1-8})$ alkyl mercapto $(C_{3-6})$ amino acid, the alkyl mercapto group and the amino group being attached to different carbon atoms, or a water-soluble salt thereof is used as the dialkyl sulfide.

**2.** A process as claimed in claim 1, characterized in that a methyl mercapto ($C_3$ or $C_4$) amino acid is used as the dialkyl sulfide.

**3.** A process as claimed in claim 1 or 2, characterized in that DL methionine is used.

**4.** A process as claimed in any of claims 1 to 4, characterized in that the initial reduction step is carried out in water and the reaction mixture obtained is subsequently reacted with the mercaptan in the presence of an organic solvent or solvent mixture, preferably at 0 to 20°C.

## Revendications

**1.** Procédé de préparation de mercaptides d'or (I) de formule générale Au-S-R, dans laquelle R est un reste organique, par réaction d'un halogénure d'or (III) ou d'un complexe halogénure d'or (III) avec un dialkylsulfure en proportion molaire de 1 à 2 ou plus, en présence d'une quantité d'eau au moins équivalente au composé Au (III), entre -20 et 40° C en formant le complexe :
halogénure d'or (I) -dialkylsulfure et le dialkylsulfoxyde, par réaction du complexe halogénure d'or (I) -dialkylsulfure entre -20 et 40° C et si besoin en présence d'un solvant organique ou d'un mélange de solvants avec un mercaptan de formule R-S-H, R ayant la même signification que dans les mercaptides d'or (I) et isolement du mercaptide d'or (I) du mélange réactionnel de la manière connue en soi, procédé caractérisé en ce qu'on utilise comme dialkylsulfure un alkyl ($C_1$ à $C_8$) mercapto [acide ($C_3$ à $C_6$) aminé], les groupes alkylmercapto et amino étant fixés sur des atomes de C différents, ou un des ses sels solubles dans l'eau.

**2.** Procédé selon la revendication 1, caractérisé en ce qu'on utilise comme dialkylsulfure un acide méthyl-mercapto- ($C_3$- ou $C_4$-) aminé.

**3.** Procédé selon la revendication 1 ou 2, caractérisé en ce qu'on utilise la DL-méthionine.

**4.** Procédé selon l'une des revendications 1 à 4, caractérisé en ce qu'on réalise d'abord l'étape de réduction dans l'eau et qu'ensuite on fait réagir le mélange réactionnel ainsi obtenu en présence d'un solvant organique ou mélange de solvants avec le mercaptan, de préférence entre 0 et 20° C.